# EUROPEAN PATENT APPLICATION

(11) **EP 2 388 592 A1**
(43) Date of publication of application: **23.11.2011**
(21) Application number: 10005215.8
(22) Date of filing: 19.05.2010
(51) Int. Cl.: G01N 33/543

(54) **Beads and process for making thereof and sensor device**

(71) Applicant: Stichting Dutch Polymer Institute, 5612 AB Eindhoven (NL)
(72) Inventor: The designation of the inventor has not yet been filed
(74) Representative: Renkema, Jaap

(57) **Abstract**

The Invention relates to a bead comprising an analyte detecting agent for interacting with an analyte and a biocompatible semi-permeable shell containing the analyte detecting agent, wherein the semi-permeable membrane is permeable to the analyte and non-permeable to the analyte detecting agent. Further, a process for making the bead is disclosed, comprising the steps of preparing a curable liquid containing the analyte and dispensing the curable liquid in droplets Into a curing environment to cure the curable liquid to form the semi-permeable shell.

## Description

The present invention relates to beads for the analysis of a sample fluid suspected of containing one or more target analytes, such as biological compounds. The present invention also relates to a process for preparing such beads. The present invention further relates to a sensor device comprising such beads and an analyzing means for characterizing the beads to determine the presence and/or the concentration of the target analytes. The present invention further relates to a method for the analysis of a sample fluid using such beads.

Diagnostic tools for detecting and/or quantifying biological analytes are generally based on specific binding interactions between a ligand and a receptor (*i.e.,* a specific binding pair). Specific binding pairs used commonly in diagnostics include antigen-antibody, hormone-receptor, drug-receptor, cell surface antigen-lectin, biotin-avidin, and complementary nucleic acid strands. The analyte for detection may be either member of the specific binding pair; alternatively, the analyte may be a ligand analog that competes with the ligand for binding to the complementary receptor.

One known type of a biosensor is a chip-based sensor where analyte detecting agents are immobilized on the surface of the chip substrate. A biosensor based on an enzyme immobilized on an electrode is known e.g. from US5,770,439. In this patent publication, a conductivity biosensor is disclosed comprising a plurality of substrate layers pressed together In a vertical stack. Each substrate layer bears a conductive electrode formed thereon by screen printing a thick film paste. Each electrode has immobilized thereon an enzyme for sensing an analyte. Such biosensors are in general suitable for use for analysis *in vitro,* but are less suitable for in *vivo* applications.

Bead-assisted analyte detection is known from WO2008/111013. This patent publication discloses beads for use in bio-sensing, the bead comprising an organic moiety, and one or more magnetic or magnetisable particles. The bead is lyophilized. The presence of an analyte in a sample fluid is analyzed by contacting a sample fluid with the beads and detecting the interaction between the sample fluid and the organic moiety. This publication further discloses a method for manufacturing the lyophilized bead. The method comprises providing a liquid mixture having a freezing temperature, said liquid mixture comprising one or more magnetic or magnetisable particles and an organic moiety, atomizing or spraying the liquid mixture into a fluid medium having a temperature lower than said freezing temperature, thus forming a bead, and freezing drying the bead.

There is growing demand for sensors such as chemosensors and biosensors which are modular, highly sensitive, easily deployable and which can be used in a wide range of applications.

An objective of the present invention Is to provide a bead which allows sensing analytes In a wide range of applications.

Accordingly, one aspect of the present invention provides a bead comprising an analyte detecting agent for interacting with an analyte and a biocompatible semi-permeable shell containing the analyte detecting agent, wherein the semi-permeable membrane is permeable to the analyte and non-permeable to the analyte detecting agent.

The term "analyte", as used herein, refers to a substance whose presence, absence, or concentration is to be determined according to the present invention. Typical analytes may include, but are not limited to, hydronium ions, organic molecules, metabolites such as glucose or ethanol, proteins, peptides, nucleic acid segments, molecules such as pharmaceuticals, antibiotics or drugs, drugs of abuse, molecules with a regulatory effect in enzymatic processes such as promoters, activators, inhibitors, cofactors, DNA, RNA, antigens, and fragments and products thereof, or any other substance for which attachment sites, binding members or receptors (such as antibodies) can be developed.

The term "analyte detecting agent", as used herein, refers to a molecule or material capable of generating a detectable signal or capable of binding to another molecule or forming a complex which generates a detectable signal. Suitable analyte detecting agents for use in different detection systems and methods of the present invention are numerous, and are extensively described in the art. These may be optical agents such as chromophores, fluorophores, phosphorophores, near infrared dyes, chemiluminescent-, electrochemiluminescent-, or biochemiluminescent agents, radioactive labels, magnetic and/or electric labels, enzymes, specifically bondable ligands, non-radioactive nuclear contrast agents (*i.e.* molecules containing an abundance of atoms with a high nuclear diameter, such as iodides), microbubbles detectable by sonic resonance, and the like. Analyte detecting agents can be directly detected by a sensor. Alternatively, the analyte detecting agents can become detectable after a subsequent development process. Typically, the analyte detecting agents used in the methods of the present invention is analyte-specific, i.e. capable of binding specifically to the analyte.

The bead according to the present invention can function as a free-standing sensor, which can be directly placed *in situ* in an environment where a sample fluid containing analytes is present. While the biocompatible shell is In contact with a sample fluid, *e.g.* blood in a blood vessel, to detect the concentration of certain analytes, the analytes enter the bead through the semi-permeable shell and interact with the analyte detecting agent. The detecting agent does not directly contact with the outside environment, since It Is trapped in the shell. The interaction between the analyte and the analyte detecting agent may cause changes in the conditions of the analyte detecting agent which generate signals detectable by suitable detecting means. The detection of the interaction between an analyte and an analyte detecting agent is well-known to the skilled person, and therefore are not described in detail here. Suitable detection systems include systems which use ultrasound and electromagnetic wave.

It is an advantage of the bead according to the present invention that the analysis may be performed not only *in vitro* but also *in vivo.* This is due to the fact that the analyte detecting agents, which may be deleterious if In direct contact with humans and animals especially In high concentrations, are contained within a shell which is biocompatible. Furthermore, the bead according to the present invention is advantageous not only in the case of an *in vivo* analysis, but in cases of both an *in vivo* analysis and an *in vitro* analysis. Addition of the beads to the fluid sample to be measured does not result in contamination of the fluid sample by the analyte detecting agent, since the shell prevents the analyte detecting agent from mixing in the sample liquid. If desired, the beads can be separated from the fluid sample by suitable means, such as by sieving, or micromanipulation after analysis. The beads as separated may be used for further testing.

A further advantage of the bead according to the present invention is that the concentration of the analyte detecting agent in the bead may be high. This allows use of a smaller amount of analyte detecting agent for detection of the interaction between the analyte and the analyte detecting agent, compared to a case where the analyte detecting agent is added to the sample liquid without the presence of the shell. The bead according to the invention is especially advantageous when the analyte detecting agent is toxic and/or costly. For example, many chemoresponslve materials are toxic in high concentrations. In the case of a bead, the concentrations can be as high as needed for the measurement, without contaminating the sample. Likewise, since the volume of the beads is quite small, highly expensive materials can be used in a small portion. The concentration of the analyte detecting agent with respect to the bead may be in the range of 0.00001 - 50 wt%. The preferred concentration of the analyte detecting agent depends on the specific analyte detecting agent and analyte. The concentration may be at least 0.00005 wt%, at least 0.0001 wt%, at least 0.0005 wt%, at least 0.001 wt%, at least 0.005 wt%, at least 0.1 wt%, at least 0.5 wt%, at least 1 wt%, at least 5 wt%, or at least 10 wt%. The concentration may be at most 40 wt%, at most 30 wt%, at most 25 wt%, at most 20 wt% or at most 15 wt%. Different types of analyte detecting agents may be present In one shell, provided that these analyte detecting agents which are not spatially isolated from each other do not interfere with precision of the respective intended analysis.

Preferably, the beads have a symmetrical or near symmetrical shape. Such a shape makes the beads easier to be characterized by the detecting means, since their geometry affords an isotropic signal. The beads may have a spherical, near spherical or elliptical shape. It may also be droplet shaped. The largest dimension of the bead is herein called the diameter of the bead. The diameter of the beads may be determined e.g. by optical microscopy, light scattering spectroscopy, scanning electronic microscopy etc. Preferably, the bead has a diameter as determined by an optical microscopy as follows. Preferably, the bead has a diameter of at least 1 µm. More preferably, the bead has a diameter of at least 5 µm. Preferably, the bead has a diameter of at most 360 µm. More preferably, the bead has a diameter of at most 250 µm, more preferably 150 µm, more preferably 100 µm, more preferably 50 µm, more preferably 30 µm, more preferably 20 µm. A particularly preferred range of the diameter is 5-15 µm. For a set of beads comprising a plurality of the beads according to the invention, the diameter is preferably highly uniform and statistically monodisperse. Preferably, the average diameter of the beads Is 1-250 µm and the set has a standard deviation of at most 20% of the average diameter, preferably 10%, more preferably 5%, even more preferably 1%, most preferably 0.1 %

Preferably, the shell comprises a hydrogel. Preferred examples of a hydrogel include polyuronic acids, such as alginates. For example, if sodium alginate solutions are ink-jet printed into a bath containing calcium ions, the solution will undergo a process, whereby sodium is replaced by calcium, yielding an ionically-crosslinked hydrogel. Alginate is a linear copolymer composed (1-4)-linked *β*-D-mannuronic acid and α-L-guluronic acid, and can be easily transformed into a gel by binding the guluronic acids with a divalent cation, such as calcium (see: Biomedical microdevices 2007, 9(6) 855-862.)

Still other preferred hydrogels are non-crosslinked polyacrylamides, polyacrylic acid and Its salt and their block copolymers. These polymers are soluble at lower pH values and are Insoluble at higher pH values.

Still other preferred hydrogels are selected proteins which are gelatin and/or collagen and which is/are added as aqueous solutions to the liquid of polymerisable monomers. These proteins are classic examples of materials capable of forming thermally reversible hydrogels, where polymer chains become entangled, but which can be reversed by heating.

In further embodiments of the present invention, the shell Is non-degradable. By non-degradable, it is herein understood to mean that the shell maintains its state so that the analyte detecting agents are not released. Hence a non-degradable shell includes a non-reversible hydrogel. Such beads are suitable for use in environments where it is desirable that the analyte detecting agents remain separated from the sample fluid to be analyzed during the period of time the beads are In contact with the sample fluid.

In some embodiments of the present invention, the shell is biodegradable and the analyte detecting agent Is non-toxic. The shell may degrade or decompose in other ways in the sample and the analyte detecting agent is released. The bead may further contain medicament. In this case, the beads may be taken in by the human body and the signals generated by the analyte detecting agent may be detected by a remote detector. When the shell degrades, the medicament is released to the outer environment. The analyte detecting agents are no longer concentrated and the signal is no longer detectable. Thus, by measuring the signal (or the disappearance thereof), it can be determined whether the medicament has been delivered to desired locations.

In some embodiments of the present invention, the bead further comprises other components such as reference dyes and/or pigments. Other additional components are materials which would allow for remote sensing. For example, suspensions of certain nanoparticles yield a concentration-dependent signal when measured by ultrasound by loading the beads with a high concentration of contrast agent, the presence and integrity of the beads may be remotely monitored *In vivo*. Further examples of the additional components for similar applications include near-infrared quantum dots, spin-labelled components for ESR measurements, and the like.

In some embodiments of the present invention, the bead further comprises at least one magnetic or magnetisable particle. This allows for remote manipulation of the system. The manipulation of beads comprising magnetic or magnetisable particle is described in WO2008/111013, which is Incorporated herein by reference.

The present invention further relates to a set of beads comprising a plurality of the beads according to the present Invention. In some embodiments of the present invention, the set comprises a first group of beads and a second group of beads, wherein the analyte detecting agent of the first group of beads is different from the analyte detecting agent of the second group of beads. This allows multiple separate measurements to be taken simultaneously in the same fluid environment. The shell separating each bead from each other allows mixing of two different types of beads In one composition to be used for multiple analysis without the signals required for each analysis interfering with those from others. For example, multiple different fluorescent-labeled antibodies Incorporated in different beads can be used simultaneously for analyzing one sample. Different types of beads may be distinguishable by their sizes. When using sizes of the beads for distinguishing the types of the beads, their size distributions preferably has substantially no overlap. Also, the sizes are preferably monodisperse. A further advantage of these embodiments is that the multiple separate measurements are provided in a much more flexible manner compared to prior art chip-based sensors where the layout of the chip substrate must be specifically designed for different analyte detecting agents. In the chip-based sensors, change in the target analytes requires change In the chip substrate design. According to the beads composition according to the present invention, changing of the analyte target can be accomplished easily by changing the type of the beads in the composition.

The beads may be brought into contact with the sample fluid to be analyzed by directly adding the beads to the sample fluid. Alternatively, the beads may be included In a fluid, granulated or included In a capsule. Other suitable methods are known to facilitate the handling of the beads. Accordingly the present invention further relates to beads contained within a matrix. For example, a suspension containing the beads according to the present invention may be provided, and the suspension can be added to the sample fluid. Preferably, the suspension comprising the beads Is biocompatible. In one embodiment, the suspension may be orally taken In by a living organism requiring analysis of certain analytes in the body. Same may be done with a dispersion containing the beads according to the present invention. Various administration methods of the suspension of the beads to a living organism are possible, including injection in vein and through eyes. Beads may be administered by inhaling if in suitable form such as a powder. Beads In a capsule may be taken in orally or rectally. An ointment comprising the beads according to the invention is also possible.

In some embodiments of the present invention, the beads are embedded in a polymer matrix. The polymer matrix is preferably biocompatible. The polymer matrix may be an artificial tissue implant. The beads provide monitoring of the interior environment of the artificial tissue implant also after the implant is within the human body. For example, oxygen content within the core of the implant may be detected while the implant is still in the body. This allows a remote detection of signals from an otherwise difficult to access environment.

The beads may be fixed In the polymer matrix. The fixation of the beads allows an easier detection of the chemical signal (e.g. oxygen content within the core of an implanted matrix) remotely from an otherwise difficult to access environment.

According to another aspect of the invention, a sensor device is provided comprising the bead according to the present invention and an analyzing means for characterizing the beads to determine the presence and/or the concentration of the target analytes.

According to another aspect of the invention, a process for making the bead according to the Invention is provided. The process comprises the steps of preparing a curable liquid containing the analyte and dispensing the curable liquid in droplets into a curing environment to cure the curable liquid to form the semi-permeable shell.

In some embodiments of the present invention, the curable liquid comprises a solution of a hydrogel in a fluid state. The curing environment comprises a liquid medium which gelates the curable liquid. Upon entering the liquid medium, crosslinking of the droplets of the solution is induced and a hydrogel is formed in which the analyte detecting agents are entrapped.

Preferably, the dispensing of the curable liquid is performed by ink-jet technology. These droplets may be ejected using several different ink-jet techniques, including both drop-on-demand techniques (e.g. thermo and piezoelectric-driven) as well as continuous jetting techniques (e.g. electrospraylng and other methods based on Rayleigh jet breakup). An advantage of this is that, due to the uniformity of the droplet sizes and volumes afforded by Inkjet printing, the resulting structures are highly reproducible and can be standardized. Once cured, the individual droplets form separate gel bead.

Said drop-on-demand technique implies ejecting a discrete amount of liquid onto a specific location. An Important feature of such drop-on-demand technique for the process of this invention is its ability to eject uniform droplets. Any jetting technique can be used that allows control of the droplet size. Examples of drop-on-demand techniques are drop-on-demand inkjet techniques, such as thermal, piezoelectric, electrostatic or acoustic inkjet techniques, or other techniques for creating microdroplets, such as pneumatic microvalve technology, double emulsion-solvent evaporation method or other emulsion methods. These technologies are known to those skilled in the art and are published, for example, in Tissue Engineering (2008). 14(1), 41-48. Pharm. Res. 8: 713-720 (1991) and Drug Development and industrial Pharmacy (2001), 27(8), 825-829. Besides these methods continuous inkjet techniques can be used, for example binary deflection-, multiple deflection-, Hertz- and microdot-inkjet techniques. A preferred example of an applicable technique for step ii) is ink-jet printing, for example ink-jet printing using piezoelectric technique or using bubble-jet technique.

Ink-jet printing is a preferred drop-on-demand method in the manufacturing process of this invention. This technique allows with high precision to rapidly dispense small aliquots of liquid. Size and shape of the droplets formed may be tuned by selecting the desired nozzle size, ink viscosity and droplet ejection conditions.

One advantage of the process of this invention is that beads with uniform diameter or a narrow distribution of diameters can be created. It is for example possible to prepare a set of beads having an average diameter of 1-250 µm having a standard deviation of at most 20% of the average diameter, preferably 10%, more preferably 5%, even more preferably 1%, most preferably 0.1%

In one embodiment of the present invention, the beads of the present invention is prepared as follows: A solution or dispersion of analyte detecting agents In sodium alginate is prepared. The sodium alginate solution Is driven Into a vibrating dispenser head. As the vibrating jet is ejected from the nozzle, it breaks up into a stream of droplets, which are collected In a calcium chloride hardening bath.

According to a yet further aspect of the present invention, a process for analyzing the presence or concentration of an analyte in a sample fluid is provided. The process comprises the steps of contacting the sample fluid with one or more beads according to the present invention and detecting the interaction between the sample fluid and the analyte detecting agent In the beads.

The invention is hereinafter illustrated by the following non-limitlng example.

### Example

5 mg of nanoparticles of alginate modified with fluorescein isothiocyanate and sulforhodamine was added to 1 ml of distilled water. The suspension obtained was added to 9 mL of 1 wt% sodium alginate solution. (See: S. Hornig, C. Biskup, A. Graefe, J. Wotschadlo, T. Liebert, G. J. Mohr, T. Heinze, Soft Matter 2008, 4, 1169) This suspension was filtered using a 1.2 µm pore size nylon membrane syringe filter (33 mm diameter), and used as such. This was loaded into the reservoir In 10 mL allquots, and dispensed into a 50mL volume of filtered hardening solution of 15 wt-% CaCl₂. positioned at a distance of 90 cm. The dispensing was performed using a Dropjet vibration-assisted continuous inkjet system manufactured by Microdrop Technologies GmbH (Norderstedt, Germany), fitted with a 100 µm inner diameter nozzle. The pressure was 400 kPa, and the frequency was 80,000 Hz, resulting in the production of dispersions of calcium alginate beads.

Aliquots of these beads were then analyzed using low magnification inverted light microscopy. Using an image analysis software, the images were analyzed to determine the bead sizes and distributions, which were found to have an average diameter of 245 µm.

To demonstrate the responsiveness of the beads to changes In the environment, fluorescence measurements were taken on a Zeiss 510 LSM inverted laser scanning microscope. The beads were rinsed with deionized water. One sample of rinsed beads was treated with a standard buffer solution having pH 4. Another sample of rinsed beads was treated with a standard buffer solution having pH of 7.

For each of the two samples, the ratio of the fluorescence signal at 520 nm from an excitation wavelength of 489 nm to the fluorescence signal at 580 nm from an excitation wavelength of 543 nm was determined. It was shown that the ratio was lower for the sample treated with the buffer solution of pH 4 compared to the sample treated with the buffer solution of pH 7.

It was confirmed that the beads made according to the present invention change their status differently depending on the condition of the outside environment, and thus the condition of the outside environment may be analyzed using the beads according to the present Invention.

It is understood that the term "comprising" is used in the present description and claims does not exclude other elements or steps. In particular, it is understood that the bead and the bead composition may further comprise other elements suitable for each situation. Where an indefinite or definite article is used when referring to a singular noun *e.g.* "a" or "an", "the", this includes a plural of that noun unless something else is specifically stated.

## Claims

1. A bead comprising an analyte detecting agent for interacting with an analyte and a biocompatible semi-permeable shell containing the analyte detecting agent, wherein the semi-permeable membrane is permeable to the analyte and non-permeable to the analyte detecting agent.

2. The bead according to claim 1 having a symmetrical or near symmetrical shape.

3. The bead according to claim 1 or 2 having an average diameter of 1-350 µm.

4. The bead according to any one of claims 1-3, wherein the shell comprises a hydrogel.

5. The bead according to any one of claims 1-4, wherein the concentration of the analyte detecting agent relative to the bead is 0.00001-50 wt%.

6. The bead according to any one of claims 1-5, wherein the shell is non-biodegradable.

7. The bead according to any one of claims 1-5, wherein the shell is biodegradable, the analyte detecting agent is non-toxic and the bead further contains a medicament.

8. A set of beads comprising a plurality of the beads according to any one of claims 1-7, wherein the average diameter of the beads is 1-250 µm and has a standard deviation of at most 20% of the average diameter, preferably 10%, more preferably 5%, even more preferably 1%, most preferably 0.1%.

9. A set of beads comprising a plurality of the beads according to any one of claims 1-7, comprising a first group of beads and a second group of beads, wherein the analyte detecting agent of the first group of beads is different from the analyte detecting agent of the second group of beads.

10. Polymer matrix comprising the beads according to any one of claims 1-7.

11. A sensor device comprising the bead according to any one of claims 1-7 and an analyzing means for characterizing the beads to determine the presence and/or the concentration of the target analytes.

12. A process for making the bead according to any one of claims 1-7, comprising the steps of preparing a curable liquid containing the analyte and dispensing the curable liquid In droplets Into a curing environment to cure the curable liquid to form the semi-permeable shell.

13. The process according to claim 12, wherein the curable liquid comprises a solution of a hydrogel in a fluid sol state, and the cuing environment comprises a liquid medium which gelates the curable liquid.

14. The process according to claim 13, wherein the dispensing of the curable liquid involves ink-jet technology.

15. A process for analyzing the presence or concentration of an analyte in a sample fluid, comprising the steps of contacting the sample fluid with one or more beads according to any one of claims 1-7 and detecting the interaction between the sample fluid and the analyte detecting agent in the beads.
